# EUROPÄISCHE PATENTSCHRIFT

(11) **EP 1 641 904 B1**
(45) Veröffentlichungstag und Bekanntmachung des Hinweises auf die Patenterteilung: **02.04.2008**
(21) Anmeldenummer: 04741677.1
(22) Anmeldetag: 28.05.2004
(51) Int. Cl.: C11D 1/835, A61K 8/30, B01F 17/36

(54) **O/W-EMULGATOR, O/W-EMULSION UND DEREN VERWENDUNGEN**
O/W EMULSIFIER, O/W EMULSION AND APPLICATIONS THEREOF
EMULSIFIANT HUILE-EAU, EMULSION HUILE-EAU ET LEURS UTILISATIONS

(30) Priorität: 26.06.2003 DE 10328686; 14.07.2003 DE 10331760
(43) Veröffentlichungstag der Anmeldung: 05.04.2006
(73) Patentinhaber: Symrise GmbH & Co. KG, 37603 Holzminden (DE)
(72) Erfinder: SCHRÖDER, Bernd, 37603 Holzminden (DE)
(74) Vertreter: Eisenführ, Speiser & Partner
(86) Internationale Anmeldenummer: PCT/EP2004/050961
(87) Internationale Veröffentlichungsnummer: WO 2004/112731

(56) Entgegenhaltungen:
- WO-A-99/37282
- PROSPERIO G ET AL: "Neuere essbare O/W-Emulgator-Mischungen" RIECHSTOFFE AROMEN KOSMETICA (RAK), FACHVERLAG V. FRANKENSTEIN. ESCHERSHAUSEN, DE, Bd. 28, Nr. 1, 1978, Seiten 8-12, XP002112591

## Beschreibung

Die Erfindung betrifft einen O/W-Emulgator, eine hieraus gefertigte O/W-Emulsion sowie deren Verwendungen.

Als das größte Organ des Menschen übt die menschliche Haut zahlreiche lebenswichtige Funktionen aus. Mit durchschnittlich etwa 2 m² Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) zu stärken oder wiederherzustellen sowie ihre Hornschicht bei auftretenden Schäden in ihrem natürlichen Regenerationsvermögen zu unterstützen. Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen. Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z. B. Kosmetikverordnung, Lebensmittel- und Arzneimittelgesetz).

Unter Emulsionen versteht man heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasserin-Öl-Emulsion (W/O-Emulsion, z. B. Butter) handelt es sich um das umgekehrte Prinzip, wobei der Grundcharakter hier durch das Öl bestimmt wird. Natürlich ist dem Fachmann eine Vielzahl von Möglichkeiten bekannt, stabile O/W-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind. Diese Systeme enthalten in der Regel Emulgatoren.

Emulgatoren sind Hilfsmittel zur Herstellung und zur Stabilisierung von Emulsionen, die in der Regel als ölige bis wachsartige, aber auch pulverförmige Stoffe vorliegen. Zur Stabilisierung von Emulsionen über einen längeren Zeitraum werden Emulgatoren benötigt, welche die Entmischung der beiden Phasen, z.B. Öl und Wasser, zum thermodynamischstabilen Endzustand unterbinden bzw. so lange verzögern, bis die Emulsion ihre Bestimmung erfüllt hat.

Emulgatoren setzen die Grenzflächenspannung zwischen den beiden Phasen herab und erreichen neben der Verringerung der Grenzflächenarbeit auch eine Stabilisierung der gebildeten Emulsion. Emulgatoren stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird. Sowohl Elastizität als auch Viskosität der Grenzflächenfilme sind wichtige Faktoren der Emulsionsstabilisierung und werden stark vom Emulgator beeinflusst. Die Stabilisierung einer bereits gebildeten Emulsion ist die wichtigste Eigenschaft der Emulgatoren und bedeutsamer als die Erleichterung der primären Verteilung der Phasen, da hierfür mechanische Hilfsmittel in ausreichendem Maße zur Verfügung stehen. Die wichtigsten Anforderungen an Emulgatoren sind:
a) Der Emulgator muss sich an der Grenzschicht zwischen den Phasen anreichern. Dazu muss er über grenzflächen- bzw. oberflächenaktive Eigenschaften verfügen, d.h. die Grenzflächenspannung der nichtmischbaren Phasen reduzieren.
b) Der Emulgator muss ferner entweder die Teilchen aufladen, so dass sie sich gegenseitig abstoßen oder eine stabile, vielfach hochviskose oder sogar feste Schutzschicht um die Teilchen bilden.

Diese Eigenschaften reichen bereits für viele Anwendungen aus. Für die Herstellung besonders langzeitstabiler Emulsionen muss das Aufrahmen oder Sedimentieren der dispergierten Teilchen jedoch verhindert und deren Neigung zum Zusammenfließen noch weiter herabgesetzt werden. Dies erreicht man durch Viskositätserhöhung der äußeren Phase und/oder die Ausbildung von schützenden viskosen Strukturen, z.B. von flüssigkristallinen oder Gelphasen. Das Emulgatorsystem muss in diesem Fall zusätzlich zum eigentlichen Emulgator noch eine weitere Komponente enthalten, die als Co-Emulgator, Stabilisator oder je nach Wirkmechanismus auch als Konsistenzgeber oder Schutzkolloid bezeichnet wird.

Damit Verbindungen als Emulgator wirksam sein können, müssen sie eine bestimmte Molekül-Struktur aufweisen. Strukturelles Kennzeichen solcher Verbindungen ist ihr amphiphiler Molekül-Aufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe). Bei der polaren Gruppe handelt es sich um eine funktionelle Gruppe, deren E-lektronenverteilung dem Molekül ein beträchtliches Dipolmoment verleiht. Diese Gruppe bedingt die Affinität zu polaren Flüssigkeiten, insbesondere die Affinität zu Wasser, und den hydrophilen Charakter der Verbindung. Aus diesem Grund wird die polare Funktion auch als hydrophile Gruppe bezeichnet.

Die apolare Gruppe ist hingegen der Teil des Moleküls, dessen Elektronenverteilung keinen nennenswerten Beitrag zum Dipolmoment leistet. Der apolare Rest bedingt die Affinität zu apolaren Flüssigkeiten, insbesondere organischen Lösungsmitteln geringer Polarität, weshalb diese Funktion auch als lipophile Gruppe bezeichnet wird. Die gemeinsame Anwesenheit hydrophiler und lipophiler Gruppen im Molekül ermöglicht Emulgatoren das Eingehen von Wechselwirkungen, sowohl mit hydrophilen als auch mit lipophilen Phasen. An der Grenzfläche tritt dadurch eine Orientierung ein, die Voraussetzung für die Grenzflächenaktivität solcher Verbindungen ist.

Ein entscheidendes Merkmal für die Charakterisierung von Emulgatoren ist das Verhältnis von hydrophilen zu lipophilen Anteilen im Molekül, ausgedrückt als hydrophiles-lipophiles Gleichgewicht (HLB-System), zu deren Bestimmung es viele experimentelle und theoretischmathematische Methoden gibt, die insgesamt auf einer Bestimmung des Verhältnisses von hydrophilen zu lipophilen Gruppen im Molekül-Anteil beruhen. Emulgatoren mit hohem HLB-Wert ergeben O/W-Emulsionen, solche mit niederem HLB-Wert bilden bevorzugt W/O-Emulsionen.

Aus dem Stand der Technik sind allerdings nur wenige O/W-Emulgatoren bekannt, die so dünnflüssig O/W-Emulsionen bilden, dass diese beispielsweise sprühbar wären. Sie beinhalten zumeist Polyethylenglykole. Zudem haben dünnflüssige O/W-Emulsionen des Standes der Technik häufig den Nachteil, dass sie instabil, auf einen engen Anwendungsbereich oder eine begrenzte Einsatzstoffauswahl begrenzt sind. Dünnflüssige, kaltherstellbare O/W-Emulsionen, in denen beispielsweise stark polare Öle - wie die in handelsüblichen Produkten sonst häufig verwendeten Pflanzenöle - ausreichend stabilisiert sind, gibt es daher zur Zeit auf dem Markt nicht. O/W-Emulsionen mit einer geringen Viskosität, die eine Lagerstabilität aufweisen, wie sie für marktgängige Produkte gefordert wird, sind nach dem Stand der Technik nur sehr aufwendig zu formulieren. Dementsprechend ist das Angebot an derartigen Formulierungen äußerst gering. Gleichwohl könnten derartige Formulierungen dem Verbraucher bisher nicht gekannte kosmetische Leistungen bieten.

Aus der EP 1 049 452 ist eine dünnflüssige kosmetische oder dermatologische O/W-Emulsion bekannt, die einen oder mehrere partiell neutralisierte Ester von Monoglyceriden und/oder Diglyceriden gesättigter Fettsäuren mit Zitronensäure und ein oder mehrere Fettalkohole gewählt aus der Gruppe der verzweigten und unverzweigten Alkylalkohole mit 12 bis 40 Kohlenstoffatomen enthält.

Die US 2003/0012801 offenbart eine spezielle Emulsion, die ein oder mehrere partiell neutralisierte Ester von Monoglyceriden und/oder Diglyceriden gesättigter Fettsäuren mit Zitronensäure, Cyclodextrinen und Retinoiden enthält.

Aus der WO 99/62468 ist ein Haarpflegemittel bekannt, das neben einem speziellen Pentaerythritolester-Öl eine zweite Ölkomponente enthält. Beispielhaft für die zweite Ölkomponente werden u.a. Zitronensäureesteröle nach einer sehr allgemein gehaltenen Formel mit ungesättigten Alkylresten zwischen 1 bis 30 Kohlenstoffatomen beansprucht. Konkrete Verbindungen dieser Substanzklasse werden nicht aufgeführt.

Der vorliegenden Erfindung liegt insbesondere die Aufgabe zugrunde, einen O/W-Emulgator zur Verfügung zu stellen, der die Nachteile des Standes der Technik überwindet.

Diese Aufgabe wird durch den O/W-Emulgator mit dem in Anspruch 1 genannten Merkmalen sowie die dazu gehörigen Verwendungen gelöst.

Erfindungsgemäß enthält der O/W-Emulgator
(a) 70 bis 90 Gew.% Glyceryloleatcitrat und
(b) 10 bis 30 Gew.% eines Viskositätsmodifizierers mit einer Viskosität im Bereich von 1 bis 10.000 mPas, dadurch gekennzeichnet, dass der Viskositätsmodifizierer ein natives Öl ist.

Der erfindungsgemäße O/W-Emulgator zeichnet sich vornehmlich aus durch:
- Herstellbarkeit von O/W Emulsionen im heiß/heiß-, heiß/kalt- und insbesondere im kalt/kalt-Verfahren,
- Bildung thermostabiler Emulsionen bei bereits niederer Einsatzkonzentration von z.B. kleiner 2 %,
- seine multivaltene Einsetzbarkeit sowohl für höherviskose Cremes, mittelviskose Milchen und Lotionen, als auch für dünnflüssige, sprühfähige Lotionen,
- eine verbesserte Dispergierung von Feststoffen in Emulsionssystemen gegenüber bekannten O/W-Emulgatoren,
- die Erhöhung der Verteilbarkeit von eingearbeiteten Feststoffen gegenüber bekannten O/W-Emulgatoren,
- ein weitestgehend pH-unabhängiges Verhalten der damit formulierten Rezepturen,
- seine Verwendbarkeit sowohl in Formulierungen mit polaren als auch unpolaren Ölen,
- seine Kompatibilität mit Hydrogelbildnern und Hydrokolloiden,
- die Abwesenheit von Polyethylenglykolen
- seine Kombinierbarkeit mit Co-Emulgatoren und konsistenzregulierenden Komponenten und
- seine Kombinierbarkeit mit UV-A und UV-B-Filtern zur Verbesserung der Wasserhaftigkeit von Sonnenschutzprodukten.

Mit dem Emulgator gefertigte O/W-Emulsionen zeichnen sich vornehmlich aus durch:
- Herstellung mit geringem Energieaufwand: die Wasserphase oder sogar Wasser- und Ölphase (bei geeigneter Wahl der weiteren Komponenten) können kalt verarbeitet werden,
- eine gute Viskositätsstabilität,
- eine hohe pH-Stabilität,
- eine gute Temperaturstabilität,
- sehr feine und homogene Emulsionsstrukturen mit glänzender Oberfläche,
- problemlose Einarbeitung von polaren und unpolaren Ölen, wobei die Einarbeitung des O/W-Emulgators wahlweise über die Wasserphase oder die Ölphase erfolgen kann und
- kaufmännische sowie logistische Vorteile hinsichtlich der Beschaffung und Lagerhaltung eines einzigen O/W-Emulgator für Lotionen und Cremes.

Vorzugsweise ist das Glyceryloleatcitrat ein Ester von Mono- und/oder Dioleinsäureglyceriden mit Zitronensäure, wobei der Ester insbesondere durch Veresterung von Zitronensäure mit Monooleinsäureglycerid im Molverhältnis von 0,3:1 bis 1,5:1, bevorzugt 0,7:1 bis 1,2:1, erhältlich ist. Glyceryloleatcitrat mit den vorgenannten Eigenschaften verbessert insbesondere die Stabilität des O/W-Emulgators und hierauf basierender Emulsionen.

Ferner ist bevorzugt, dass das Glyceryloleatcitrat durch Neutralisation einen pH-Wert im Bereich von 5 bis 8, insbesondere 5,8 bis 6,2, insbesondere von 6,0, besitzt (gemessen jeweils in 10%iger 1:1-Wasser/Methanol-Mischung). Besonders bevorzugt sind voll neutralisierte Ester. Zur Neutralisation eignet beispielsweise Natriumhydroxid.

Das Glyceryloleatcitrat des erfindungsgemäßen O/W-Emulgators weist vorzugsweise einen HLB-Wert im Bereich von 9 bis 15, insbesondere 11 bis 13 auf.

Ferner ist bevorzugt, wenn der Anteil des Glyceryloleatcitrats, d.h. der Komponente (a), am O/W-Emulgator im Bereich von 75 bis 85 Gew.%, insbesondere bei 80 Gew.%, liegt.

Mit den vorgenannten Modifikationen kann die Langzeitstabilität des O/W-Emulgators deutlich verbessert und die Herstellung stabiler O/W-Emulsionen unterstützt werden.

Weiterhin ist bevorzugt, dass die Viskosität des Viskositätsmodifizierers im Bereich von 1 bis 1.000 mPas, besonders bevorzugt 1 bis 100 mPas, liegt. Durch Einhaltung der genannten Bereichsgrenzen kann die Rheologie des O/W-Emulgators so eingestellt werden, daß er bei Raumtemperatur fließfähig ist. Dies ermöglicht die Kaltherstellbarkeit von O/W Emulsionen. Die Viskositätsangaben gelten jeweils für 25°C und sind mit einem Brookfield-Viskosimeter (Modell RV) mit einer für den jeweiligen Viskositätsbereich sinnvollen Spindel und bei einer für die Messung angemessenen Umdrehungszahl ermittelt worden. Es wurde die Viskosität der reinen, erfindungsgemäßen Viskositätsvermittler bestimmt, wobei anstelle des angegebenen Viskosimeters auch andere, für die Messung der dynamischen Viskosität geeignete Viskosimeter Einsatz finden können, da sie in der Regel nur ein geringfügig abweichendes Ergebnis liefern.

Erfindungsgemäß ist der Viskositätsmodifizierer ein natives Öl, insbesondere Capryl- / Capric-Triglycerid (CAS Registry No. 65381-09-1). Letzterer Viskositätsmodifizierer liegt als Blend vor und die im Triglycerid enthaltenden Fettsäuren weisen vorzugsweise einen Anteil der Caprylsäure im Bereich von 50 bis 72 Gew.% und einen Anteil der Capricsäure im Bereich von 26 bis 45 Gew.% auf. Gerade die Wahl von Capryl- /Capric-Triglycerid als Viskositätsmodifizierer hat einen überraschend positiven Effekt sowohl auf die Stabilität des O/W-Emulgators als auch die damit zubereiteten Emulsionen. Capryl- / Capric-Triglycerid ist beispielsweise bei der Henkel KGaA, Deutschland unter dem Handelsnamen Myritol oder bei der Symrise GmbH & Co. KG, Deutschland unter der Bezeichnung Neutralöl (2/950160) erwerbbar.

Kosmetische und dermatologische Zusammensetzungen in Form einer Emulsion enthalten vorzugsweise einen Anteil des erfindungsgemäßen O/W-Emulgators an der Gesamtformulierung im Bereich von 2 bis 5 Gew.%. Bei Verwendung von Co-Emulgatoren in Zusammensetzungen vorgenannten Typs liegt deren Anteil an der Gesamtformulierung vorzugsweise bei 1 bis 10 Gew.%. Gegebenfalls anwesende Stabilisierungsmittel haben vorzugsweise einen Anteil an der Gesamtformulierung von 0,1 bis 5 Gew.%.

Ferner wurde gefunden, dass Glyceryloleatcitrat oder der O/W-Emulgator in einer Emulsion allein oder zusammen mit anderen kosmetischen Hilfsstoffen folgende Wirkung hat:

Steigerung des Sonnenschutzfaktors von UV-Filtern (UVA und/oder UVB Schutz); Stabilisierung von UV-Filtern (verbesserte Photostabilisation); Verbesserung der Löslichkeit und/oder Suspension von festen UV-Filtern; Erhöhung der Wasserfestigkeit von Sonnenschutzprodukten; Unterstützung bei der Bildung einer Gelnetzwerkstruktur; Steigerung des Wirksamkeit von aktiven Stoffen, wie z.B. Antioxidantien, Konservierungsmittel, Aufheller (Skin Lightener) und Bräunungsmittel, Parfümöle, Chelatbildner; Steigerung des Substantivität von aktiven Wirkstoffen auf der Haut und/oder dem Haar; Verbesserung der Verteilung von kosmetischen Ölen (Pflanzenöle, Mineralöle, Emollients), Wirkstoffen, Vitaminen, Parfümölen und ätherischen Ölen auf der Haut; Unterstützung einer gleichmäßigen Verteilung von Repellent-Wirkstoffen; Beitrag zu einer optimalen Verteilung von Konservierungssystemen in der Wasserphase; Unterstützung die Barriere-Funktion der Haut; Reduktion der Agglomerationsrate von anorganischen UV-Filtern (Titandioxid, Zinkoxid) und Farbpigmenten; Unterstützung der Verteilung von Aluminiumsalzen in Antiperspirant-Produkten; Verträglichkeit mit Alkoholen, auch mit Ethanol; Verbesserte Stabilisierung von Emulsionen als Haupt- oder Coemulagator.

Formulierungsbeispiele: Hautpflegecreme (O/W), Body-Lotion, Sonnenschutzcreme (O/W), Sonnenschutzmilch (O/W), sprühbare Sonnenmilch (O/W), Sensitiv Balsam Roll-on und Creme (O/W), sprühbare Deo-Lotion, Antiperspirant-Lotion, Haarkurspülung, Haargel-Wax for men, Haarcreme, Getönte Tagescreme, Mascara, Pflegelotion für Feuchttücher.

Glyceryloleatcitrat oder der O/W-Emulgator können zusammen mit Lichtschutzmitteln formuliert werden. Geeignete Lichtschutzmittel sind z.B. organische UV-Absorber aus der Klasse der 4-Aminobenzösäure und Derivate, Salicylsäure-Derivate, Benzophenon-Derivate, Dibenzoylmethan-Derivate, Diphenylacrylate, 3-Imidazol-4-yl-acrylsäure und deren Ester, Benzofuran-Derivate, Benzylidenmalonat-Derivate, polymere UV-Absorber (enthaltend einen oder mehrere Silizium-organische Reste), Zimtsäure-Derivate, Campher-Derivate, Trianilino-s-TriazinDerivate, 2-Hydroxyphenylbenzotriazol-Derivate, 2-Phenylbenzimidazol-5-sulfonsäure und deren Salze, Anthranilsäurementhylester, Benzotriazolderivate.

Glyceryloleatcitrat oder der O/W-Emulgator können ferner in kosmetische und/oder dermatologische Zubereitungen eingearbeitet werden, die Pigmente, bevorzugt feinteilige Pigmente enthalten. Hierbei kann es sich um organische oder anorganische Pigmente handeln. Bevorzugtes organisches Pigment ist 2,2'-Methylen-bis-[6-(2H-benzotriazol-2-yl)-4-(1,1,3,3-tetramethylbutyl)-phenol] (Tinosorb^{®} M). Anorganische Pigmente oder Mikropigmente auf Basis von Metalloxiden und/oder anderen in Wasser schwerlöslichen oder unlöslichen Metallverbindungen sind insbesondere Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Diese Pigmente sind röntgenamorph oder nichtröntgenamorph. Besonders bevorzugt sind feinteilige Pigmente auf der Basis von TiO₂ und ZnO.

Glyceryloleatcitrat oder der O/W-Emulgator können ferner in kosmetische und/oder dermatologische Zubereitungen eingearbeitet werden, die wie üblich zusammengesetzt sind und dem kosmetischen und/oder dermatologischen Lichtschutz, ferner zur Behandlung, der Pflege und der Reinigung der Haut und/oder der Haare und als Schminkprodukt in der dekorativen Kosmetik dienen. Entsprechend können derartige Zubereitungen, je nach ihrem Aufbau beispielsweise als Hautschutzcreme, Reinigungsmilch, Sonnenschutzlotion, Nährcreme, Tages- oder Nachtcreme usw. verwendet werden. So können derartige Zubereitungen beispielsweise als Emulsion, Lotion, Milch, Creme, Hydrodispersionsgel, Balm, Spray, Schaum, Haar-Shampoo, Haar-Pflegemittel, Haar-Conditioner, Roll-on, Stick oder Make-up vorliegen.

Es ist gegebenenfalls möglich und vorteilhaft, derartige Zubereitungen als Grundlage für pharmazeutische Formulierungen zu verwenden. Bevorzugt sind insbesondere solche kosmetischen und dermatologischen Zubereitungen, die in der Form eines Hautpflege- bzw. Schminkprodukts vorliegen.

Zur Anwendung werden die so beispielhaft erwähnten kosmetischen und dermatologischen Zubereitungen in der für Kosmetika üblichen Weise auf die Haut und/oder die Haare in ausreichender Menge aufgebracht.

Die Lipidphase kann vorteilhaft gewählt werden aus folgender Substanzgruppe:

Mineralöle, Mineralwachse; natürlicher Öle wie z.B. Rizinusöl; Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; Alkylbenzoate; Silikonöle wie Dimethylpolysiloxan, Diethylpolysiloxan, Diphenylpolysiloxan sowie Mischformen daraus.

Die Lipidphase der Emulsionen, Oleogele bzw. Hydrodispersionen oder Lipodispersionen im Sinne der vorliegenden Veröffentlichung werden vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, -palmitat, -stearat, -oleat, n-Butylstearat, n-Hexyllaurat, n-Decyloleat, Isooctylstearat, Isononylstearat, Isononylisononanat, 2-Ethyl-hexylpalmitat, Ethylhexyllaurat, 2-Hexyldecylstearat, 2-Octyldodecylpalmitat, Oleyloleat, Oleylerucat, Erucyloleat sowie synthetische, halbsynthetische und natürliche Gemische solcher Ester, z.B. Jojobaöl, 2-Ethylhexyl 2-Ethylhexanoat, Cetearyl 2-Ethylhexanoat, Diisopropyl Adipat, Triisonananoin.

Ferner kann die Lipidphase vorteilhaft gewählt werden aus der Gruppe der verzweigten und unverzweigten Kohlenwasserstoffe und -wachse, der Siliconöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft im Sinne der vorliegenden Erfindung wird Cyclomethicon (Octamethylcyclotetrasiloxan) als zu verwendendes Silikonöl eingesetzt. Aber auch andere Silikonöle sind ähnlich vorteilhaft zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase von Zubereitungen im Sinne dieser Erfindung enthält gegebenenfalls vorteilhaft wasserlösliche Pflanzenextrakte, Alkohole, Diole oder Polyole (Niederalkyl), sowie deren Ether, vorzugsweise Ethanol, Isopropanol, Propylenglykol, Glycerin, Ethylenglykol-monoethyl- oder -monobutylether, Propylenglykolmonomethyl, -monoethyl- oder -monobutylether, Diethylenglykolmonomethyl- oder -monoethylether und analoge Produkte, ferner Alkohole (Niederalkyl), z.B. Ethanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxyropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen und dermatologischen Zubereitungen im Sinne dieser Erfindung können kosmetische Hilfsstoffe enthalten, wie sie üblicherweise in solchen Zubereitungen verwendet werden, z.B. Konservierungsmittel, Antioxidantien, Vitamine, Bakterizide, Parfüme, Substanzen zum Verhindern des Schäumens, Farbstoffe, Pigmente, die eine färbende Wirkung haben, Verdickungsmittel, Tenside, Emollients, Emulgatoren, anfeuchtende und/oder feuchthaltende Substanzen, Feuchtigkeitsvermittlern, Fette, Öle, Wachse, Pflanzenextrakte oder andere übliche Bestandteile einer kosmetischen oder dermatologischen Formulierung wie Alkohole, Niederalklyalkohole, Polyole, Niederalkylpolyole, Polymere, Schaumstabilisatoren, Komplexbildner, Elektrolyte, organische Lösungsmittel, Treibgase, Silikone oder Silikonderivate.

Die jeweils einzusetzenden Mengen an kosmetischen oder dermatologischen Hilfs- und Trägerstoffen und Parfüm können in Abhängigkeit von der Art des jeweiligen Produktes vom Fachmann leicht ermittelt werden.

Ein zusätzlicher Gehalt an Antioxidantien ist im allgemeinen bevorzugt. Als günstige Antioxidantien können alle für kosmetische und/oder der matologische Anwendungen geeignete oder gebräuchliche Antioxidantien verwendet werden.

Die Menge der Antioxidantien (eine oder mehrere Verbindungen) in den Zubereitungen beträgt vorzugsweise 0,001 bis 30 Gew.%, besonders bevorzugt 0,05 bis 20 Gew.%, insbesondere 1 bis 10 Gew.%, bezogen auf das Gesamtgewicht der Zubereitung.

Vorteilhaft werden die Antioxidantien aus der folgenden Gruppe gewählt: Aminosäuren (z.B. Glycin, Histidin, 3,4-Dihydroxyphenylalanin, Tyrosin, Tryptophan) und deren Derivate, Imidazole (z.B. Urocaninsäure) und deren Derivate, Peptide (D,L-Carnosin, D-Camosin, L-Carnosin, Anserin) und deren Derivate, Carotinoide, Carotine (z.B. alpha-Carotin, beta-Carotin, Lycopin) und deren Derivate, Chlorogensäure und deren Derivate, Liponsäure und deren Derivate, Aurothioglucose, Propylthiouracil und andere Thiole (z.B. Thioredoxin, Glutathion, Cystein, Cystin, Cystamin und deren Glycosyl- und N-Acylderivate oder deren Alkylester) sowie deren Salze, Dilaurylthiodipropionat, Distearylthiodipropionat, Thiodipropionsäure und deren Derivate sowie Phenolsäureamide phenolischer Benzylamine (z.B. Homovanillinsäure-, 3,4-Dihydroxyphenylessigsäure-, Ferulasäure-, Sinapinsäure-, Kaffeesäure-, Dihydroferulasäure-, Dihydrokaffeesäure-, Vanillomandelsäure- oder 3,4-Dihydroxymandelsäureamide des 3,4-Dihydroxybenzyl-, 2,3,4-Trihydroxybenzyl- bzw. 3,4,5-Trihydroxybenzylamins), Catecholoxime oder Catecholoximether (z.B. 3,4-Dihydroxybenzaldoxim oder 3,4-Dihydroxybenzaldehyd-O-ethyloxim), 2-Hydrazino-1,3-thiazole und Derivate, ferner (Metall-)chelatoren (z.B. 2-Hydroxyfettsäuren, Phytinsäure, Lactoferrin), Huminsäure, Gallensäuren, Gallenextrakte, Bilirubin, Biliverdin, Folsäure und deren Derivate, Ubichinon und Ubichinol und deren Derivate, Vitamin C und dessen Derivate (z.B. Ascorbylpalmitat, Magnesiumascorbylphosphat, Ascorbylacetat), Tocopherole und Derivate (z.B. Vitamin-E-Acetat), Vitamin A und Derivate (z.B. Vitamin-A-Palmitat), Rutinsäure und deren Derivate, Flavonoide (z.B. Quercetin, alpha-Glucosylrutin) und deren Derivate, Phenolsäuren (z.B. Gallussäure, Ferulasäure) und deren Derivate (z.B. Gallussäurepropylester, -ethylester, -octylester), Furfurylidenglucitol, Dibutylhydroxytoluol, Butylhydroxyanisol, Harnsäure und deren Derivate, Mannose und deren Derivate, Zink und dessen Derivate (z.B. ZnO, ZnSO₄), Selen und dessen Derivate (z.B. Selenomethionin), Stilbene und deren Derivate (z.B. Stilbenoxid, Resveratrol).

Ebenfalls vorteilhafte Antioxidantien sind beschrieben in EP-A 900781, EP-A 1 029 849, EP-A 1 066 821, WO-A 01/43712, WO-A 01/70176, WO-A 01/98235 oder auch in WO-A 01/98258.

Sofern Vitamin E und/oder dessen Derivate das oder die Antioxidantien darstellen, ist vorteilhaft, deren jeweilige Konzentrationen aus dem Bereich von 0,001 bis 10 Gew.%, bezogen auf das Gesamtgewicht der Formulierung, zu wählen.

Sofern Vitamin A, bzw. Vitamin-A-Derivate, oder Carotine bzw. deren Derivate das oder die Antioxidantien darstellen, ist vorteilhaft, deren jeweilige Konzentrationen aus dem Bereich von 0,001 bis 10 Gew.%, bezogen auf das Gesamtgewicht der Formulierung, zu wählen.

Es folgen zehn beispielhafte Formulierungen, in denen der erfindungsgemäße O/W-Emulgator als Dracorin GOC bezeichnet wird. Dracorin GOC enthält 70-90 Gew.% Glyceryloleatcitrat und 10-30 Gew.% Capryl-/ Capric-Triglycerid. Bezogen auf die im Capryl- / Capric-Triglycerid enthaltenden Fettsäuren liegt ein Anteil der Caprylsäure bei 50 bis 65 Gew.% und ein Anteil der Capricsäure bei 35 bis 45 Gew.%. Das Capryl- / Capric-Triglycerid hat eine Viskosität von ca. 30 mPas. Der O/W-Emulgator hat einen pH-Wert von ca. 6 gemessen in 10%iger 1:1-Wasser/Methanol-Mischung und einen HLB-Wert von ca. 12.

Beispiel (1): kaltherstellbare Bodylotion (O/W), Beispiele (2) und(4): Sonnenschutz-milch (O/W) und Beispiel (3): Gesichtscreme (O/W) mit Sonnenschutz;

| | Name | INCI Name | (1) | (2) | (3) | (4) |
|---|---|---|---|---|---|---|
| A | Abil 100 | Dimethicone | | | 0.3 | |
| | Cetiol OE | Dicaprylyl Ether | | 5.0 | 1.5 | |
| | Copherol 1250 | Tocopheryl Acetate | | 0.5 | 0.5 | 0.5 |
| | Corapan TQ® | Diethylhexyl 2,6-naphthalate | | 5.0 | 5.0 | 2.5 |
| | Cutina FS 45 | Palmitic Acid (and) Stearic Acid | | 2.0 | | |
| | Cutina MD | Glyceryl Stearate | | 1.0 | 2.0 | |
| | DC 345 | Cyclomethicone | 2.0 | | | |
| | Dracorin GOC 2/008580 | Glyceryl Oleate Citrate (and) Caprylic/Capric Triglyceride | 2.0 | 4.0 | 5.0 | 4.0 |
| | Dragoxat EH 2/044115 | 2-Ethylhexyl 2-Ethylhexanoate | | | 1.5 | |
| | Edeta BD | Disodium EDTA | | | | 0.1 |
| | Hostacerin DGMS | Polyglyceryl-2 Stearate | | | | 4.0 |
| | Isopropylpalmitat | Isopropylpalmitat | 7.0 | | | |
| | Keltrol T | Xanthan Gum | | | | 0.4 |
| | Lanette 16 | Cetyl Alcohol | | | 1.5 | |
| | Lanette O | Cetearyl Alcohol | | 1.0 | | |
| | Miglyol 812 | Caprylic/Capric Triglyceride | | | | 5.0 |
| | Neo Heliopan® 357 | Butyl Methoxydibenzoylmethane | | 0.8 | 2.0 | 1.5 |
| | Neo Heliopan® HMS | Homosalate | | | 5.0 | 8.0 |
| | Neo Heliopan® MBC | 4-Methylbenzylidene Camphor | | 3.0 | | |
| | Neo Heliopan® OS | Ethylhexyl Salicylate | | | 5.0 | |
| | PCL Liquid 100 2/066240 | Cetearyl 2-Ethylhexanoate | 8.0 | 5.0 | | |
| | Prisorine 3505 | Isostearic Acid | | | | 0.5 |
| | SF 1214 | Cyclopentasiloxane (and) Dimethicone | | | | 1.0 |
| | Solbrol P | Propylparaben | | 0.1 | 0.1 | 0.1 |
| | Tegosoft TN | C12-15 Alkyl Benzoate | | | | 4.0 |
| | Trilon BD | EDTA | | | 0.1 | |
| | Zinc Oxide neutral H&R | Zinc Oxide | | | | 7.0 |
| | | | | | | |
| B | 1,3 - Butylenglykol | Butylene Glycol | | 3.0 | | |
| | Carbopol 1382 | Carbomer | 0.2 | | | |
| | Carbopol ETD 2050 | Carbomer | | 0.2 | 0.3 | |
| | Glycerin 99 % | Glycerin | | | 3.0 | 4.0 |
| | Karion F | Sorbitol | 5.0 | | | |
| | Keltrol T | Xanthan Gum | | 0.2 | 0.5 | |
| | Lanette E | Sodium Cetearyl Sulfate | | | | 0.75 |
| | Natronlauge, 10% aq. | Sodium Hydroxide | 0.2 | | | 2.5 |
| | Dragocid Liquid 2/060140 | Phenoxyethanol, Methyl-, Ethyl-, Butyl-, Propyl-, Isobutylparaben | 0.8 | | | |
| | Neo Heliopan® AP | Disodium Phenyl Dibenzimidazole Tetrasulfonate | | | | 2.2 |
| | Neo Heliopan® AP, 10%ige Lösung neut. Mit NaOH | Disodium Phenyl Dibenzimidazole Tetrasulfonate | | 22.0 | 25.0 | |
| | Phenoxyethanol | Phenoxyethanol | | 0.7 | 0.7 | 0.7 |
| | Solbrol M | Methylparaben | | 0.2 | 0.2 | 0.2 |
| | Wasser, dest. | Water (Aqua) | 74.5 | 43.5 | 36.9 | 50.55 |
| | | | | | | |
| C | Natronlauge, 10% aq. | Sodium Hydroxide | | 2.4 | 3.5 | |
| | Perfume Oil | Fragrance (Parfum) | | | | 0.5 |
| | | | | | | |
| D | Parfümoel | Fragrance (Parfum) | 0.3 | 0.3 | 0.3 | |
| | Alpha-Bisabolol nat. | Bisabolol | | 0.1 | 0.1 | |
| | | | | | | |
| | (Alle Angaben in %) | Summe | 100 | 100 | 100 | 100 |

| | | | | | | |
|---|---|---|---|---|---|---|
| Zum Beispiel (1) - Herstellungsverfahren (alle Schritte bei Umgebungstemperatur): Teil A/C und B getrennt einwiegen. Carbopol mit Ultra Turrax eindispergieren, dann neutralisieren. Teil B zu Teil A/C unter Verwendung des Ultra-Turrax geben und dann emulgieren. Zu den Beispielen (2), (3) - Herstellungsverfahren: Teil A auf ca. 85°C erhitzen. Für Teil B Rohstoffe ohne Carbopol und Keltrol einwiegen. Carbopol und Keltrol mit Ultra Turrax eindispergieren. Auf ca. 85°C erhitzen. Teil B zu Teil A geben. Teil C sofort zu A/B geben und sodann heiß homogenisieren (Ultra Turrax). Unter Rühren abkühlen lassen und Teil D zugeben und verrühren. Zum Beispiel (4) - Herstellungsverfahren: Teil A auf ca. 85°C erhitzen (ohne Keltrol und Zinkoxid). Keltrol und Zinkoxid mit dem Ultra Turrax in die heiße Lipidphase eindispergieren. Teil B auf ca. 85°C erhitzen. Teil B zu Teil A geben. Unter Rühren auf 60°C abkühlen und homogenisieren (Ultra Turrax). Anschließend unter Rühren auf Raumtemperatur abkühlen lassen. Teil C zugeben und homogenisieren. | | | | | | |

Beispiel (5): Haargel-Wax for Men DGHST 0086/01; Beispiel (6): Haarcreme (O/W) DCHST 0087/00; Beispiel (7): Haarkurspülung (O/W) mit Dragoderm DLHCR 0088/00; Beispiel (8): Sensitiv Balsam Roll-on (O/W) DRDEO 0089/00; Beispiel (9): Pflegelotion für Feuchttücher (O/W) DDTSS 0091/00; Beispiel (10): Hautpflegecreme (O/W) DCSKN 0092/00

| | Rohstoffe | INCI-Bezeichnung | (5) | (6) | (7) | (8) | (9) | (10) |
|---|---|---|---|---|---|---|---|---|
| A | Abil 350 | Dimethicone | | | | | | 1.5 |
| | Abil B 8852 | Dimethicone Copolyol | | 1.0 | | | | |
| | Cetiol HE | PEG-7 Glyceryl Cocoate | 1.0 | | | | | |
| | Cutina HR Plv. | Hydrogenated Castor Oil | | | 0.5 | | | |
| | Dracorin GMS 2/008474 | Glyceryl Stearate | | | 3.0 | 2.0 | | 1.0 |
| | Drago-Oat-Active 2/060900 | Water (Aqua), Butylene Glycol, Avena Sativa (Oat) Kernel Extr. | | | | | 1.0 | |
| | Dragoxat EH 2/044115 | Ethylhexyl Ethylhexanoate | | | | | | 7.0 |
| | Dracorin GOC 2/008580 | Glyceryl Oleate Citrate (and) Caprylic/Capric Triglyceride | 5.0 | | 3.0 | 3.0 | | 4.0 |
| | Eumulgin B2 | Ceteareth-20 | | | | 2.0 | | |
| | Farnesol 2/027040 | Farnesol | | 0.1 | | | | |
| | Fitoderm | Vegetable squamane | | | | | | 3.0 |
| | Lanette 16 | Cetyl Alcohol | | | | 2.5 | | 4.0 |
| | Lanette O | Cetearyl Alcohol | | 4.0 | 1.5 | | | |
| | Lösungsvermittler 2/014170 | PEG-40 Hydrogenated Castor Oil, Trideceth-9, Water (Aqua) | 15.0 | | | | | |
| | Neo-Dragocid Flüssig 2/060110 | Triethylene Glycol, Imidazolidinyl Urea, Methylparaben, Propylparaben, Dehydroacetic Acid | | | | | 0.4 | |
| | Neutralöl 2/950161 | Caprylic/Capric Triglyceride | 10.0 | | | | | |
| | PCL Liquid 100 2/066240 | Cetearyl Ethylhexanoate | 5.0 | 2.0 | 0.5 | 1 | | |
| | Pemulen TR-2 | Acrylates/C10-30 Alkyl Acrylate Crosspolymer | | | | | 0.2 | |
| | Rewoderm LI 520-70 | PEG-200 Hydrogenated Glyceryl Palmate | 1.5 | | | | | |
| | Varisoft BT 85 | Behentrimonium Chloride | | 1.0 | | | | |
| | Varisoft TA 100 | Distearyldimonium Chloride | | | 2.0 | | | |
| | Wasser | Water (Aqua) | | | | | 76.6 | |
| | | | | | | | | |
| B | -(-Alpha-) Bisabolol, nat. 2/012685 | Bisabolol | | | | | 0.1 | |
| | Aloe Vera-Gel-Konz. 10/1 2/912800 | Water (Aqua), Aloe Barbadensis Gel | | | | 1.0 | | |
| | Butylenglykol | Butylene Glycol | 1.0 | | | | | |
| | Citronensäure, 10%ig in Wasser | Citric Acid | 0.3 | | | | | |
| | Dragocid Liquid 2/060140 | Phenoxyethanol, Methyl-, Ethyl-, Butyl-, Propyl-, Isobutylparaben | | | 0.8 | 0.8 | | 0.8 |
| | Dragoxat EH 2/044115 | Ethylhexyl Ethylhexanoate | | | | | 8.0 | |
| | Dracorin GOC | Glyceryl Oleate Citrate (and) Caprylic/Capric Triglyceride | | 2.0 | | | 0.8 | |
| | Glycerin 99.5 P | Glycerin | | 6.0 | | | | 3.0 |
| | Glydant Plus Liquid | DMDM Hydantoin, lodopropynyl Butylcarbamate | 0.2 | | | | | |
| | Keltrol F | Xanthan Gum | | | | | | 0.25 |
| | Paraffinöl 5 Gr.E | Paraffinum Liquidum | | | | | 8.3 | |
| | PCL Liquid 100 2/066240 | Cetearyl Ethylhexanoate | | | | | 3.9 | |
| | Wasser | Water (Aqua) | 60.8 | 82.7 | 84.7 | 85.7 | | 75.15 |
| | | | | | | | | |
| C | Deolite 2/027095 | Pentylene Glycol, Dimethyl Phenylpropanol | | | | 1.0 | | |
| | Dragocid Liquid 2/060140 | Phenoxyethanol, Methylparaben, Ethylparaben, Butylparaben, Propylparaben, Isobutylparaben | | 0.8 | | | | |
| | Dragoderm 2/012550 | Glycerin, Triticum Vulgare (Wheat) Gluten, Water (A- | | | 3.5 | | | |
| | | qua) | | | | | | |
| | NaOH 10% Lsg. | Sodium Hydroxide | | | | | 0.4 | |
| | Parfümöl | Fragrance | 0.2 | 0.4 | 0.5 | 1.0 | | |
| | | | | | | | | |
| D | Parfümöl | Fragrance | | | | | 0.3 | 0.3 |
| | | | | | | | | |
| | (Alles in %) | Summe | 100 | 100 | 100 | 100 | 100 | 100 |

| | | | | | | | | |
|---|---|---|---|---|---|---|---|---|
| Zum Beispiel (5) - Herstellungsverfahren: Phasen A und B getrennt voneinander auf ca. 75°C erhitzen. Unter moderatem Rühren zusammengeben bis der Gel-Wax homogen ist. Anschließend abkühlen lassen, Phase C bei ca. 40°C zugeben und bis zur Homogenität einrühren. pH: ca. 5.2. Zum Beispiel (6) - Herstellungsverfahren: Alle Rohstoffe der Phase A mischen, auf 80°C erhitzen und mit einem Ultra Turrax homogenisieren. Mit einem Blattrührer kaltrühren, wobei die Rührgeschwindigkeit mit abnehmender Temperatur reduziert wird. Phase C bei ca. 35°C zugeben. pH: ca. 5.9. Zu den Beispielen (7) und (8) - Herstellungsverfahren: Phasen A und B getrennt voneinander auf ca. 80°C erhitzen. Phase B zu A geben (Ultra-Turrax) und emulgieren. Mit einem Blattrührer kaltrühren, dabei die Rührgeschwindigkeit mit abnehmender Temperatur reduzieren. Phase C bei ca. 30°C zugeben. pH: ca. 4.2 für (7) und 5.2 für (8). Zu den Beispielen (9) und (10) - Herstellungsverfahren: Pemulen TR-2 bzw. Keltrol F in Wasser unter einem Ultra-Turrax quellen. Phasen A und B getrennt voneinander auf ca. 80°C erhitzen. Phase B zu A geben (Ultra-Turrax) und emulgieren.Phase C zugeben und nochmals homogenisieren. Mit einem Blattrührer kaltrühren, dabei die Rührgeschwindigkeit mit abnehmender Temperatur reduzieren. Phase D bei ca. 35°C zugeben. pH: ca. 5.5 für (9) und 5.2 für (10). | | | | | | | | |

## Patentansprüche

1. O/W-Emulgator enthaltend
70 bis 90 Gew.% Glyceryloleatcitrat und
10 bis 30 Gew.% eines Viskositätsmodifizierers mit einer Viskosität im Bereich von 1 bis 10.000 mPas, **dadurch gekennzeichnet, dass** der Viskositätsmodifizierer ein natives Öl ist.

2. O/W-Emulgator nach Anspruch 1, **dadurch gekennzeichnet, dass** das Glyceryloleatcitrat ein Ester von Mono- und/oder Dioleinsäureglyceriden mit Zitronensäure ist.

3. O/W-Emulgator nach Anspruch 2, **dadurch gekennzeichnet, dass** das Glyceryloleatcitrat durch Veresterung von Zitronensäure mit Monooleinsäureglycerid im Molverhältnis von 0,3:1 bis 1,5:1 erhältlich ist.

4. O/W-Emulgator nach Anspruch 3, **dadurch gekennzeichnet, dass** das Molverhältnis im Bereich von 0,7 :1 bis 1,2:1 liegt.

5. O/W-Emulgator nach Anspruch 1, **dadurch gekennzeichnet, dass** das Glyceryloleatcitrat voll neutralisiert ist.

6. O/W-Emulgator nach Anspruch 1, **dadurch gekennzeichnet, dass** das Glyceryloleatcitrat einen pH-Wert im Bereich von 5 bis 8 hat.

7. O/W-Emulgator nach Anspruch 6, **dadurch gekennzeichnet, dass** der pH-Wert im Bereich von pH 5,8 bis pH 6,2 liegt.

8. O/W-Emulgator nach Anspruch 7, **dadurch gekennzeichnet, dass** der pH-Wert 6,0 beträgt.

9. O/W-Emulgator nach Anspruch 1, **dadurch gekennzeichnet, dass** das Glyceryloleatcitrat einen HLB-Wert im Bereich von 9 bis 15 hat.

10. O/W-Emulgator nach Anspruch 9, **dadurch gekennzeichnet, dass** der HLB-Wert im Bereich von 11 bis 13 liegt.

11. O/W-Emulgator nach Anspruch 1, **dadurch gekennzeichnet, dass** der Anteil des Glyceryloleatcitrat am O/W-Emulgator im Bereich von 75 bis 85 Gew.% liegt.

12. O/W-Emulgator nach Anspruch 11, **dadurch gekennzeichnet, dass** der Anteil des Glyceryloleatcitrats am O/W-Emulgator 80 Gew.% beträgt.

13. O/W-Emulgator nach Anspruch 1, **dadurch gekennzeichnet, dass** die Viskosität des Viskositätsmodifizierers im Bereich von 1 bis 1.000 mPas liegt.

14. O/W-Emulgator nach Anspruch 13, **dadurch gekennzeichnet, dass** die Viskosität des Viskositätsmodifizierers im Bereich von 1 bis 100 mPas liegt.

15. O/W-Emulgator nach Anspruch 1, **dadurch gekennzeichnet, dass** der Viskositätsmodifizierer ein Capryl- / Capric-Triglycerid ist.

16. O/W-Emulgator nach Anspruch 15, **dadurch gekennzeichnet, dass** ein Anteil der Caprylsäure bei 50 bis 72 Gew.% und ein Anteil der Capricsäure bei 26 bis 45 Gew.% jeweils bezogen auf die im Triglycerid enthaltenden Fettsäuren liegt.

17. Verwendung von Glyceryloleatcitrat oder eines O/W-Emulgators nach einem oder mehreren der Ansprüche 1 bis 16 in kosmetischen oder dermatologischen Zusammensetzungen.

18. Verwendung nach Anspruch 17 zur Komplexierung von Metallspuren in lipophilen Systemen, als Synergist und Lösungsvermittler für Antioxidantien oder in Hautpflege- oder Schminkprodukten.

19. Verwendung nach Anspruch 17, **dadurch gekennzeichnet, dass** die kosmetische oder dermatologische Zusammensetzung eine Emulsion ist und ein Anteil des O/W-Emulgators an der Gesamtformulierung 2 bis 5 Gew.% beträgt.

20. O/W-Emulsion enthaltend einen O/W-Emulgator nach einem oder mehreren der Ansprüche 1 bis 16.

## Claims

1. O/W emulsifier, containing
70 to 90 % by weight glyceryloleate citrate and
10 to 30 % by weight of a viscosity modifier with a viscosity in the range from 1 to 10,000 mPas, **characterised in that** the viscosity modifier is a native oil.

2. O/W emulsifier according to Claim 1, **characterised in that** the glyceryloleate citrate is an ester of mono- and/or diolenic acid glycerides with citric acid.

3. O/W emulsifier according to Claim 2, **characterised in that** the glyceryloleate citrate is obtainable by the esterification of citric acid with mono-oleic acid glyceride in a molar ratio from 0.3:1 to 1.5:1.

4. O/W emulsifier according to Claim 3, **characterised in that** the molar ratio lies in the range from 0.7:1 to 1.2:1.

5. O/W emulsifier according to Claim 1, **characterised in that** the glyceryloleate citrate is fully neutralised.

6. O/W emulsifier according to Claim 1, **characterised in that** the glyceryloleate citrate has a pH value in the range from 5 to 8.

7. O/W emulsifier according to Claim 6, **characterised in that** the pH value lies in the range from pH 5.8 to pH 6.2.

8. O/W emulsifier according to Claim 7, **characterised in that** the pH value is 6.0.

9. O/W emulsifier according to Claim 1, **characterised in that** the glyceryloleate citrate has an HLB value in the range from 9 to 15.

10. O/W emulsifier according to Claim 9, **characterised in that** the HLB value lies in the range from 11 to 13.

11. O/W emulsifier according to Claim 1, **characterised in that** the portion of glyceryloleate in the O/W emulsifier lies in the range from 75 to 85 % by weight.

12. O/W emulsifier according to Claim 11, **characterised in that** the portion of glyceryloleate in the O/W emulsifier amounts to 80 % by weight.

13. O/W emulsifier according to Claim 1, **characterised in that** the viscosity of the viscosity modifier lies in the range from 1 to 1,000 mPas.

14. O/W emulsifier according to Claim 13, **characterised in that** the viscosity of the viscosity modifier lies in the range from 1 to 100 mPas.

15. O/W emulsifier according to Claim 1, **characterised in that** the viscosity modifier is a caprylic/capric triglyceride.

16. O/W emulsifier according to Claim 15, **characterised in that** a portion of the caprylic acid lies at 50 to 72 % by weight and a portion of the capric acid lies at 26 to 45 % by weight, related in each case to the fatty acids contained in the triglyceride.

17. Use of glyceryloleate citrate or of an O/W emulsifier according to one or more of Claims 1 to 16 in cosmetic or dermatological compositions.

18. Use according to Claim 17 for complexing metal traces in lipophilic systems, as a synergist and a solubilizer for antioxidants or in skin care or make-up products.

19. Use according to Claim 17, **characterised in that** the cosmetic or dermatological composition is an emulsion and a portion of the O/W emulsifier amounts to 2 to 5 % by weight of the total formulation.

20. O/W emulsion containing an O/W emulsifier according to one or more of Claims 1 to 16.

## Revendications

1. Emulsifiant H/E comprenant
70 à 90 % en masse d'oléate-citrate de glycéryle et
10 à 30 % en masse d'un modificateur de viscosité ayant une viscosité dans le domaine de 1 à 10 000 mPa.s, **caractérisé en ce que** le modificateur de viscosité est une huile native.

2. Emulsifiant H/E selon la revendication 1 **caractérisé en ce que** l'oléate-citrate de glycéryle est un ester de mono- et/ou diglycérides d'acide oléique avec l'acide citrique.

3. Emulsifiant H/E selon la revendication 2 **caractérisé en ce que** l'oléate-citrate de glycéryle peut être obtenu par estérification de l'acide citrique avec un monoglycéride d'acide oléique dans le rapport molaire de 0,3 : 1 à 1,5 : 1.

4. Emulsifiant H/E selon la revendication 3 **caractérisé en ce que** le rapport molaire est dans le domaine de 0,7 : 1 à 1,2 : 1.

5. Emulsifiant H/E selon la revendication 1 **caractérisé en ce que** l'oléate-citrate de glycéryle est totalement neutralisé.

6. Emulsifiant H/E selon la revendication 1 **caractérisé en ce que** l'oléate-citrate de glycéryle a un pH dans le domaine de 5 à 8.

7. Emulsifiant H/E selon la revendication 6 **caractérisé en ce que** le pH est situé dans le domaine de pH 5,8 à pH 6,2.

8. Emulsifiant H/E selon la revendication 7 **caractérisé en ce que** le pH est 6,0.

9. Emulsifiant H/E selon la revendication 1 **caractérisé en ce que** l'oléate-citrate de glycéryle a une valeur HLB dans le domaine de 9 à 15.

10. Emulsifiant H/E selon la revendication 9 **caractérisé en ce que** la valeur HLB est dans le domaine de 11 à 13.

11. Emulsifiant H/E selon la revendication 1 **caractérisé en ce que** la proportion d'oléate-citrate de glycéryle dans l'émulsifiant H/E est dans le domaine de 75 à 85 % en masse.

12. Emulsifiant H/E selon la revendication 11 **caractérisé en ce que** la proportion d'oléate-citrate de glycéryle dans l'émulsifiant H/E est de 80 % en masse.

13. Emulsifiant H/E selon la revendication 1 **caractérisé en ce que** la viscosité du modificateur de viscosité est dans le domaine de 1 à 1 000 mPa.s.

14. Emulsifiant H/E selon la revendication 13 **caractérisé en ce que** la viscosité du modificateur de viscosité est dans le domaine de 1 à 100 mPa.s.

15. Emulsifiant H/E selon la revendication 1 **caractérisé en ce que** le modificateur de viscosité est un triglycéride caprylique/caprique.

16. Emulsifiant H/E selon la revendication 15 **caractérisé en ce qu'**une proportion d'acide caprylique est de 50 à 72 % en masse et une proportion d'acide caprique est de 26 à 45 % en masse, dans chaque cas par rapport aux acides gras contenus dans le triglycéride.

17. Utilisation d'oléate-citrate de glycéryle ou d'un émulsifiant H/E selon une ou plusieurs des revendications 1 à 16 dans des compositions cosmétiques ou dermatologiques.

18. Utilisation selon la revendication 17 pour la complexation de traces de métaux dans des systèmes lipophiles, comme agent de synergie et promoteur de dissolution pour antioxydants ou dans des produits pour les soins de la peau ou de maquillage.

19. Utilisation selon la revendication 17 **caractérisée en ce que** la composition cosmétique ou dermatologique est une émulsion et une proportion de l'émulsifiant H/E dans la formulation totale est de 2 à 5 % en masse.

20. Emulsion H/E contenant un émulsifiant H/E selon une ou plusieurs des revendications 1 à 16.
